# EUROPÄISCHE PATENTSCHRIFT

(11) **EP 1 885 740 B1**
(45) Veröffentlichungstag und Bekanntmachung des Hinweises auf die Patenterteilung: **02.12.2015**
(21) Anmeldenummer: 06763112.7
(22) Anmeldetag: 10.05.2006
(51) Int. Cl.: C07K 1/36

(54) **PROTEINEXTRAKTION AUS FORMALIN FIXIERTEM GEWEBE**
EXTRACTION OF PROTEINS FROM FORMALIN-FIXED TISSUE
EXTRACTION DE PROTEINES DE TISSU A FIXATION A LA FORMALINE

(30) Priorität: 19.05.2005 DE 102005023011
(43) Veröffentlichungstag der Anmeldung: 13.02.2008
(73) Patentinhaber: Qiagen GmbH, 40724 Hilden (DE)
(72) Erfinder: PORSCHEWSKI, Peter, 40764 Langenfeld (DE); BECKER, Karl-Friedrich, 82110 Germering (DE)
(86) Internationale Anmeldenummer: PCT/EP2006/062198
(87) Internationale Veröffentlichungsnummer: WO 2006/122898

(56) Entgegenhaltungen:
- WO-A-2005/116256
- IKEDA KIMIMASA ET AL: "Extraction and analysis of diagnostically useful proteins from formalin-fixed, paraffin-embedded tissue sections" JOURNAL OF HISTOCHEMISTRY AND CYTOCHEMISTRY, Bd. 46, Nr. 3, März 1998 (1998-03), Seiten 397-403, XP002396023 ISSN: 0022-1554
- KAPLAN B ET AL: "Micropurification techniques in the analysis of amyloid proteins." JOURNAL OF CLINICAL PATHOLOGY (LONDON), Bd. 56, Nr. 2, Februar 2003 (2003-02), Seiten 86-90, XP002396024 ISSN: 0021-9746

## Beschreibung

Die vorliegende Erfindung betrifft ein Verfahren, mit dem Proteine, insbesondere intakte, volle Länge Proteine aus Formalin fixierten biologischen Proben in Lösung gebracht werden und später quantifiziert werden können.

In vielen Ländern ist das Fixieren von Geweben in Formalin zur nachfolgenden histopathologischen Untersuchung Standard, um z. B. gesundes von krankem Gewebe zu unterscheiden. Formalin fixiertes und Paraffin eingebettetes (FFPE) Gewebe wird seit Jahrzehnten gesammelt und ist die Quelle von unzähligen diagnostischen Studien. Es sind Gewebe von den meisten Organen, von verschiedenen Krankheitsstadien, vor/nach/während einer Therapie usw. verfügbar. FFPE Gewebe bietet den Vorteil, dass die Morphologie sehr gut erhalten ist. Makromoleküle (DNA, RNA, Proteine) können allerdings - aufgrund der Quervernetzung - nicht mehr adäquat untersucht werden. Nach derzeitiger Meinung ist Formalin fixiertes Gewebe ungeeignet, um daraus routinemäßig und verläßlich Proteine in ausreichender Menge zur nachfolgenden Quantifizierung zu isolieren (z.B. Espina et al. 2003). Entsprechend wird nach Alternativen zur Formalin-Fixierung der Gewebe für die nachfolgende Quantifizierung von Makromolekülen gesucht. Hier sind u. a. zu nennen das Einfrieren der Gewebe (Kryo-Schnitte) und die aktuell diskutierten experimentellen Fixiermethoden wie z.B. 70%iger Äthanol (z.B. Ahram et al. 2003). Gefriermaterial ist zwar eine exzellente Quelle für Makromoleküle; die Sammlung, Prozessierung und Lagerung der Gewebe ist jedoch aufwendig. Die experimentellen Fixiermethoden sind ein guter Kompromiß zwischen erhaltener Morphologie und Intaktheit der Makromoleküle; sie spielen aber bei retrospektiven Studien keine große Rolle.

Die durch das humane Genomprojekt identifizierten Kandidatenmoleküle werden auf Proteinebene in großen retrospektiven und prospektiven Untersuchungen auf ihre klinische Verwendbarkeit geprüft werden müssen. DNA- und RNA-Analysen galten früher als nicht durchführbar an Formalin-fixiertem Material. Heute sind solche Analysen Standard, sogar nach Laser-gestützter Gewebe-Mikrodissektion. Für Proteinanalysen könnte das gleiche gelten. Die Verfahren zur Isolierung von Nukleinsäuren unterscheiden sich dramatisch von denen zur Proteinisolierung. So dürfen zur Proteinisolierung z.B. keine Proteasen verwendet werden, da dadurch Proteine angedaut und damit nicht mehr intakt sind. Das Verfahren von Expression Pathology (WO 2004/080579 A2) setzt genau an diesem Punkt an und verwendet Proteasen in Kombination mit Hitzeeinwirkung, um proteolytische Fragmente - also Peptide - zu isolieren. Diese können in nachfolgenden Schritten dann mittels Massenspektrometrie analysiert werden. Das Verfahren von Expression Pathology unterscheidet sich somit vom dem hier beschriebenen Verfahren. Protein-Vernetzungen - bedingt durch Formalin - können durch ausreichende Hitzeinwirkung gelöst werden.

Folgende Beispiele dafür sind bekannt:
(1) Chromatin-Immunpräzipitation (ChIP). Die ChIP ist eine Prozedur, mit der man nachweisen kann, ob ein Ziel-Protein an eine bestimmte DNA-Sequenz in vivo bindet. Dabei werden intakte Zellen mit Formalin fixiert, um DNA-Protein- und Protein-Protein-Vernetzungen hervorzurufen. Die Zellen werden anschließend lysiert, die DNA geschert, um sie in kleinere Stücke zu teilen. Die DNA-Protein-Komplexe werden dann mittels eines Antikörpers gegen das Zielprotein immunpräzipitiert. Die DNA-Protein-Verknüpfungen werden nun durch Hitzeeinwirkung aufgelöst und die Proteine durch Proteasen zerstört. Schließlich wird mittels Polymerase Kettenreaktion (PCR) nachgewiesen, ob eine bestimmte DNA-Sequenz durch den spezifischen Antikörper mit immunpräzipitiert wurde. Die Hitzeeinwirkung ist hier das Prinzip der Auflösung der durch Formalin ausgelösten Vernetzung.
(2) Antigenrückgewinnung. Durch Formalin-Fixierung verlieren Antigene häufig ihre Immunreaktivität. Immunhistochemische Studien mit vielen Antikörpern waren deshalb nur bedingt möglich. Heutzutage sind Methoden verfügbar, mit denen die Immunreaktivität der meisten Antigene zurückgewonnen werden kann (antigen retrieval). Das Prinzip dabei ist - nach Deparaffinierung und Rehydrierung der Gewebeschnitte - die Einwirkung von Hitze auf das Gewebe in einer wässrigen Lösung. Eine Quantifizierung der Reaktion ist bei Immunhistochemie jedoch nur bedingt möglich.

Aus der WO 2004/080579 A2 ist ein Verfahren zur Herstellung eines Biomoleküllysats bekannt bei dem Formalin fixierte Gewebeproben in einem Puffersystem erhitzt werden und danach mit einem proteolytisch wirksamen Enzym behandelt werden, um das Gewebe aufzuschließen. Ikeda et al., (1998) J Histochem Cytochem 46:397-403, offenbart ein Verfahren zur Extraktion von Proteinen aus einer Formalin-fixierten Probe mit Hilfe eines Puffersystems welches ein Detergenz enthält aber keine proteolytischwirksame Verbindung umfasst, wobei die Proben bei 100 Grad Celsius für 20 Minuten und bei 60 Grad Celsius für 2 Stunden inkubiert werden.

Es ist derzeit jedoch keine Methode verfügbar, mit der intakte Proteine aus Formalin fixierten Geweben verlässlich und mit modernen Methoden quantitativ und sensitiv untersucht werden können. Immunhistochemie (IHC), der Nachweis von Immunreaktivität und Zuordnung zu einzelnen Zellen im Gewebeschnitt, ist derzeit die einzige Methode, um Proteine an Formalin fixierten Geweben zu untersuchen. IHC ist jedoch nur bedingt quantifizierbar. Formalin-Fixierung erhält zwar exzellent die Morphologie der Gewebe, führt jedoch zur intensiven Vernetzung von Proteinen untereinander und mit anderen Makromolekülen, beispielsweise mit DNA. Bisherige Versuche zur Proteinisolierung aus Formalin fixierten Geweben (z.B. Ahram et al. 2003) scheiterten, da sie generell eine sehr schlechte Ausbeute an Proteinen bieten, unzuverlässig sind, sich lediglich auf Western Blot Untersuchungen (z.B. Ikeda et al 1998) beschränken und oft nicht zum Nachweis bzw. zur Quantifizierung von intakten Proteinen führen. Ikeda et al. beschreibt eine Methode, die zwar zur Isolierung von Proteinen aus FFPE Gewebe geeignet ist, aber nicht zu einer quantitativen Isolierung führt. Grund hierfür ist, dass offensichtlich der Inkubationsschritt bei 60°C nicht ausreicht, um die Proteine quantitativ zu isolieren. Es müssen Proteine in kleinsten fixierten Gewebeproben, z.B. aus Biopsien, quantifiziert werden können. Mit derzeitigen Verfahren gelingt es nicht eine ausreichende Proteinmenge aus solchen Proben zu gewinnen, z.B. für den Nachweis mittels Proteinarrays. Folglich sind diese Verfahren ungeeignet, um Krankheitsmarker im klinischen Alltag zu bestimmen. Mit Western blots allein kann der steigende Bedarf an der Quantifizierung von Proteinen aus Formalin fixierten Geweben weder in der Klinik noch in der Forschung gedeckt werden. Heutzutage kommt der Entwicklung von Hochdurchsatzverfahren, z.B. Proteinarrays, eine immense Bedeutung zu. Intakte Proteine aus Formalin fixierten Geweben gelten derzeit als nicht untersuchbar mit Proteinarrays (Espina et al. 2003). Aufgabe der Erfindung war es daher ein verbessertes Verfahren zur Extraktion von Proteinen aus Formalin fixierten biologischen Proben bereitzustellen.

Die Aufgabe wurde gelöst durch ein Verfahren zur quantitativen Extraktion von Proteinen aus einer Formalin fixierten biologischen Probe, wobei die die biologische Probe in einem Puffer bei einer Temperatur inkubiert wird, die ausreicht, um die Proteine freizusetzen, wobei der Puffer ein Detergenz und keine proteolytisch wirksame Verbindung enthält, und die biologische Probe im Puffer zunächst gekocht wird und dann bei einer Temperatur höher als 60 °C weiter inkubiert wird. Weiterbildungen der Erfindung ergeben sich aus den jeweiligen Unteransprüchen.

Die hier beschriebene Erfindung betrifft eine wesentliche Optimierung der Proteinextraktion aus fixierten biologischen Proben wie Geweproben zur nachfolgenden Analyse und insbesondere zur Quantifizierung der Proteine, die kompatibel zu aktuellen Hochdurchsatzmethoden, beispielsweise Proteinarrays, in der Klinik und in der experimentellen Forschung ist. Proben aus verschiedenen Krankheitsstadien oder -verläufen sind auf diese Weise zugänglich für die Analyse und Quantifizierung mittels Antikörperdetektion.

Überraschenderweise liefert das erfindungsgemäße Verfahren zur Proteinextraktion aus Formalin fixierten Geweben ein exzellentes Ergebnis und hebt die Nachteile des Standes der Technik auf. Die bisher verwendeten Techniken führten offenbar nicht zum Erfolg, weil: (a) die Proben zu kurz erhitzt wurden (z.B. nur 50 Sekunden bei 100°C); oder (b) Proteasen verwendet wurden (keine intakten Proteine isolierbar); oder (c) zwar ein Detergenz verwendet wurde, es erfolgte aber keine ausreichende Erhitzung der Proben (z.B. nicht höher als 60°C, damit eine zu geringe Proteinausbeute). Erst das hier beschriebene Verfahren, nämlich beispielsweise (a) der Einsatz eines Protease freien Puffers, (b) die Verwendung eines Detergenz UND (c) die höhere Erhitzung (wärmer als 60°C, für länger als 50 Sekunden) führte unvorhergesehener Weise zu einer ausreichenden Menge an intakten Proteinen, die anschließend genau quantifiziert werden konnten.

Weitere Nachweisverfahren der isolierten intakten Proteine können dann beispielsweise Western blot, Proteinarray, Immunpräzipitation, SELDI-TOF, Massenspektroskopie, ELISA und 2-D-Gelelektrophorese sein.

Im Folgenden sollen die Erfindung anhand von Ausführungsbeispielen weiter erläutert werden.
Fig. 1. zeigt ein Beispiel einer klinischen Anwendung des erfindungsgemäßen Verfahrens.
Fig.2 zeigt beispielhaft die verbesserte Proteinausbeute durch Inkubation der Gewebeproben bei wärmer als 60°C. Gezeigt ist die Ausbeute von Alpha-Tubulin (intaktes Protein, 50 kDa) mittels Western blot nach Inkubation der Proben bei 60°C (links) im Vergleich zu dem erfindungsgemäßen Verfahren (>60°C, z.B. 80°C, rechts).
Fig.3 zeigt die Korrelation zweier Proteine (E-cadherin, alpha-Tubulin) nach Extraktion aus Formalin fixierten Geweben.
Fig.4 zeigt die Korrelation zwischen Western blot (Nachweis der Intaktheit der Proteine, Demonstration der Antikörper-Spezifität) und der einfachsten Form eines Revers-Phasen Protein Arrays (Dot blot).
Fig.5 zeigt die Korrelation von beta-Actin nach Extraktion aus FFPE Schnitten mit Puffern, die unterschiedliche Reduktionsmittel enthalten.

Mit dem vorliegenden Verfahren können entweder ein intaktes Protein oder mehrere intakte Proteine nachgewiesen und quantifiziert werden. Proteine aus völlig verschiedenen Zellkompartimenten, wie z.B. dem Zellkern, dem Zytoplasma oder der Zellmembran, können sicher isoliert und mengenmäßig bestimmt werden. Die isolierten intakten Proteine sind verdünnbar, d.h. es können Verdünnungsreihen und damit interne Standardkurven erstellt werden. Damit kann sichergestellt werden, dass der Nachweis und die Quantifizierung der Proteine im linearen Bereich stattfindet. Die Proteine können vorher bei Bedarf mittels Western blot untersucht werden, um sicher zu stellen, dass keine Kreuzreaktionen der verwendeten Nachweismittel, z.B. Antikörper, auftreten (nur eine spezifische Bande in der richtigen Größe im Western blot). Die auf die hier beschriebene Weise isolierten und quantifizierbaren intakten Proteine ergänzen in optimaler Weise Ergebnisse von immunhistochemischen Analysen, wie sie bereits im klinischen Alltag durchgeführt werden. Damit sind erstmals die exakte und sensitive Quantifizierung von intakten Proteinen (eine Aufgabe der vorliegenden Erfindung) und die zelluläre Zuordnung von Proteinen (Immunhistochemie) im fixierten Gewebe möglich. Bekannte Krankheitsmarker, beispielsweise Her2/neu bei Brutkrebspatientinnen, können nun in nie gekannter Genauigkeit klinisch bestimmt werden. Darüber hinaus können neue Krankheitsmarker vergleichend zwischen einem gesunden und einem kranken Gewebe durch die Methode identifiziert werden, indem die isolierten intakten Proteine mit gängigen Proteinmethoden, z.B. Massenspektroskopie, analysiert werden. Tierische Gewebe können mit der vorliegenden Methode ebenfalls untersucht werden. Für viele Krankheiten des Menschen, z.B. Tumorerkrankungen, sind bereits Tiermodelle verfügbar. Die tierischen Gewebe werden typischerweise in Formalin fixiert, in Paraffin eingebettet und dann histopathologisch begutachtet. Die vorliegende Methode der Proteinisolierung kann für eine exakte, sensitive und leistungsfähige Quantifizierung, z.B. mittels Proteinarrays, bekannter und neuer Krankheitsmarker in diesen Modellen eingesetzt werden. Es ist eine weitere Aufgabe dieser Erfindung ein Anwendungspaket (einen "Kit") zur Verfügung zu stellen, mit dem intakte Proteine aus Formalin fixierten menschlichen oder tierischen biologischen Proben wie Geweben verlässlich und mit hoher Ausbeute isoliert werden können. Bestandteile dieses Anwendungspaketes sind beispielsweise mindestens (a) ein Protease freies Puffersystem und (b) ein Detergenz. Ein detailliertes Protokoll zur Proteinisolierung aus Formalin fixierten Geweben kann dem Anwendungspaket beigelegt werden.

Die vorliegende Methode kann sowohl für die klinische Forschung als auch für grundlagenorientierte Untersuchungen eingesetzt werden. Noch wichtiger ist, dass das hier beschriebene Verfahren für die Proteinisolierung aus Formalin fixierten Geweben optimal in den klinischen Alltag eingebettet werden kann. So können wesentlich genauere Diagnose- und Therapieverfahren angewendet werden.

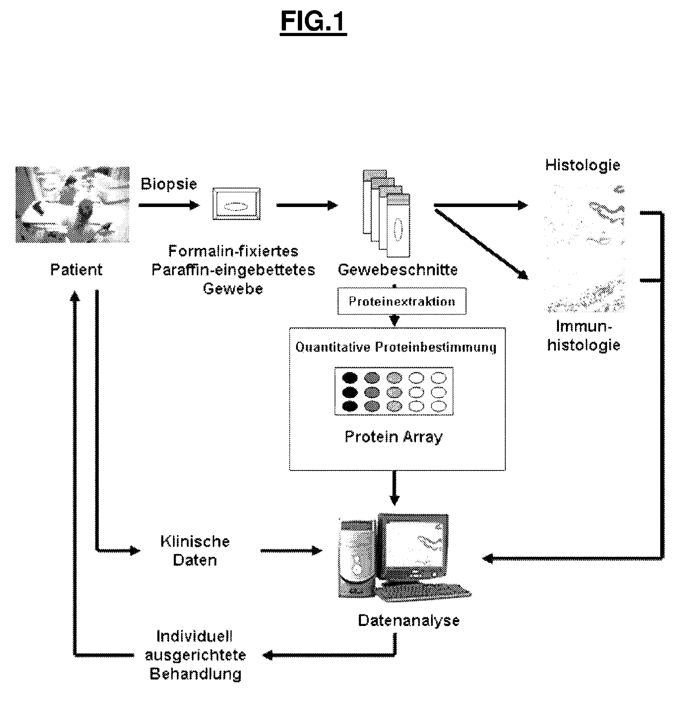

Fig. 1 zeigt ein Beispiel für die klinische Verwendung von intakten Proteinen aus routinemäßig Formalin fixierten Geweben. Damit können selbst chemisch veränderte Proteine, beispielsweise phosphorylierte oder glykosylierte Proteine, nachgewiesen werden. Bisher wird nur die Immunhistochemie zur Proteinuntersuchung am Formalin fixierten Material klinisch eingesetzt. Das vorliegende Verfahren der Proteinquantifizierung liefert ergänzende Informationen über die Expression von bekannten und neuen Krankheitsmarkern in noch nicht gekannter Genauigkeit im klinischen Alltag.

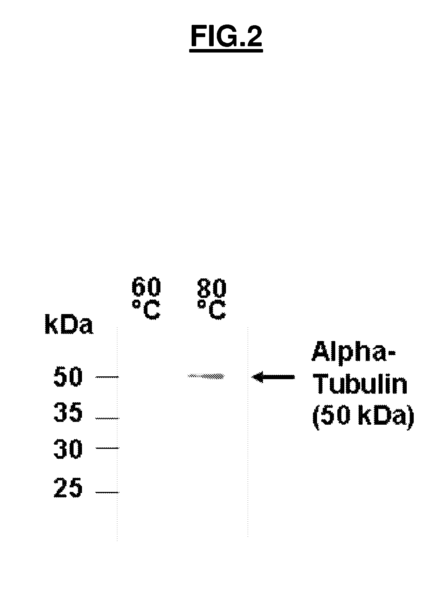

Die quantitative Proteomuntersuchung ist durch die Herstellung von Proteinarrays dabei ihren Weg in den klinischen Alltag zu finden. Formalin fixierte Gewebeproben können derzeit allerdings aufgrund der schlechten Proteinausbeute noch nicht mit diesen Hochdurchsatzmethoden untersucht werden. Das vorliegende Verfahren behebt diesen Mangel. Beispielhaft sollen hier 3 Strategien von Protein nachweisenden Mikroarrays dargestellt werden, die sich an das vorliegende Verfahren anschließen können. 1. Der sog. Sandwich Immunoarray (Antikörperarray I). Bei dieser Arrayform wird ein Protein bindendes Molekül, z.B. ein Antikörper, an einen festen Untergrund gekoppelt. Die nach dem beschriebenen Verfahren isolierten Proteine (Antigene) werden von dem Antikörper spezifisch gebunden und können dann mit einem zweiten spezifischen Antigen bindenden Antikörper, der zur Detektion markiert ist, nachgewiesen werden. 2. Antigenfänger Array (Antikörperarray II). Hier werden ebenfalls Protein bindende Moleküle, z.B. Antikörper, an einen festen Untergrund gekoppelt. Die nach der vorliegenden Methode isolierten Proteine werden mit einem Detektionsreagenz gekoppelt, z.B. einem Fluoreszenzfarbstoff, und dann erst spezifisch von den Antikörpern gebunden. Auf die Weise wird das gebundene Protein direkt nachgewiesen. Durch Vergleich von isolierten Proteinen aus verschiedenen Geweben, z.B. aus Tumorgewebe und Normalgewebe, kann durch Verwendung verschiedener Fluoreszenzfarbstoffe (z.B. Cy-3 und Cy-5) die relative Menge des interessierenden Proteins vergleichend dargestellt werden. 3. Direkter Proteinarray (Revers-Phasen Proteinarray). Bei dieser Methode werden die nach der beschriebenen Methode isolierten Proteine direkt auf eine geeignete Unterlage getropft, z.B. auf Nitrozellulose oder PVDF, und die gebundenen Proteine mit spezifischen Antikörpern direkt oder indirekt (mittels eines zweiten Detektionsantikörpers) nachgewiesen. Signalamplifikationsmethoden (z.B. CSA von DAKO) erlauben einen sehr sensitiven und spezifischen Nachweis von Proteinen in Lösung. Dieses Verfahren ähnelt sehr stark dem bekannten Dot blot. Die Erfindung stellt eine Methode für die Proteinquantifizierung aus Formalin fixierten Gewebeproben zur Verfügung. Diese Technik erlaubt erstmalig die exakte Bestimmung von Krankheitsmarkern in Gewebeproben unter kliniknahen Bedingungen. In nicht vorhersehbarer Weise wurde entdeckt, dass für eine hohe Ausbeute an intakten Proteinen aus fixierten Geweben, wie sie für die Untersuchung von kleinen Gewebeproben (z.B. Biopsien) benötigt werden, die Kombination bisher erfolgloser Techniken entscheidend ist. Bei bisherigen Verfahren werden die Gewebeproben z.B. zunächst auf 100°C erhitzt, gefolgt von einer weiteren längeren Erhitzung auf 60°C. Es wurde entdeckt, dass die weitere Erhitzung der Proben auf 60°C nicht ausreichend ist, um eine hohe Proteinausbeute zu erreichen. Eine Erhitzung der Proben auf mehr als 60°C, z.B. auf 80°C, führt jedoch zu einer wesentlich höheren Menge an gelösten Proteinen (Fig. 2). Dementsprechend kann sich das erfindungsgemäße Verfahren beispielsweise aus folgenden Schritten zusammensetzen:
(a) Blöcke aus Formalin fixierten und in Paraffin eingebetteten Gewebeproben werden geschnitten;
(b) die Schnitte werden vom Paraffin befreit;
(c) die zu untersuchenden Gewebeareale werden entweder manuell oder mittels Laser-Mikrodissektion vom Gewebeschnitt abgenommen (ggf. kann auch der komplette Gewebeschnitt untersucht werden);
(d) die abgenommenen Gewebestücke werden in einen Puffer überführt, der ein Detergenz enthält und frei von proteolytisch wirksamen Verbindungen ist. Es dürfen beispielsweise keine Proteasen, wie Trypsin oder Proteinase K, verwendet werden, um die Intaktheit der Proteine zu gewährleisten.
(e) die im Puffer befindlichen Proben werden zunächst gekocht (auf 95°C bis 100°C erhitzt). Die Inkubationszeit kann variieren, von beispielsweise 5 Minuten bis 40 Minuten. Die anzusetzende Zeit des Kochens kann beispielsweise von der Probengröße abhängen.
(f) danach werden die Proben bei einer Temperatur über 60°C (z.B. 80°C) inkubiert. Die Inkubation bei >60°C kann variieren, von beispielsweise 1 Stunde bis 6 Stunden. Die maximale Inkubationszeit bei wärmer als 60°C sollte jedoch weniger als 16 Stunden betragen.
(g) intakte Proteine sind nun in ausreichender Menge in Lösung und können beispielsweise quantifiziert werden.

In einer weiteren bevorzugten Ausführungsform des erfindungsgemäßen Verfahrens wurde ein Extraktionspuffer, der DTT enthält, verwendet. Es wurde festgestellt, dass die Verwendung von DTT als Reduktionsmittel zu einer besonders vorteilhaften Ausbeute an isoliertem, intakten Protein führte. Der Vorteil dieser Ausführungsform besteht insbesondere darin, dass die erhaltenen Lysate besonders gut direkt mit kommerziell verfügbaren und dem Fachmann bekannten Proteinquantifizierungsassays wie BioRad DC ® oder BCA-Assay ® von Pierce quantifiziert werden können und in den weiteren Analysen eingesetzt werden können. Auf ein Verdünnen der Proben kann verzichtet werden, dadurch werden Messungenauigkeiten vermieden und gewährleistet, dass in den nachfolgenden Analysen (z.B. Western Blot) gleiche Proteinmengen eingesetzt werden.

Fig. 1. zeigt ein Beispiel einer klinischen Anwendung des erfindungsgemäßen Verfahrens. Die hohe Ausbeute von intakten Proteinen aus Formalin fixierten Geweben ermöglicht erstmalig eine Integration von Histologie, Immunhistologie und quantitativer Proteinexpression am routinemäßig gewonnen Biopsie- oder Resektionsmaterial. Das Verfahren erlaubt beispielsweise die genaue Untersuchung von Krankheitsmarkern mittels Protein Arrays bei verschiedenen Krankheitsstadien (prämaligne, invasiv) oder vor und nach einer Therapie.

Fig.2 zeigt beispielhaft die verbesserte Proteinausbeute durch Inkubation der Gewebeproben bei wärmer als 60°C. Gezeigt ist die Ausbeute von Alpha-Tubulin (intaktes Protein, 50 kDa) mittels Western blot nach Inkubation der Proben bei 60°C (links) im Vergleich zu dem erfindungsgemäßen Verfahren (>60°C, z.B. 80°C, rechts).

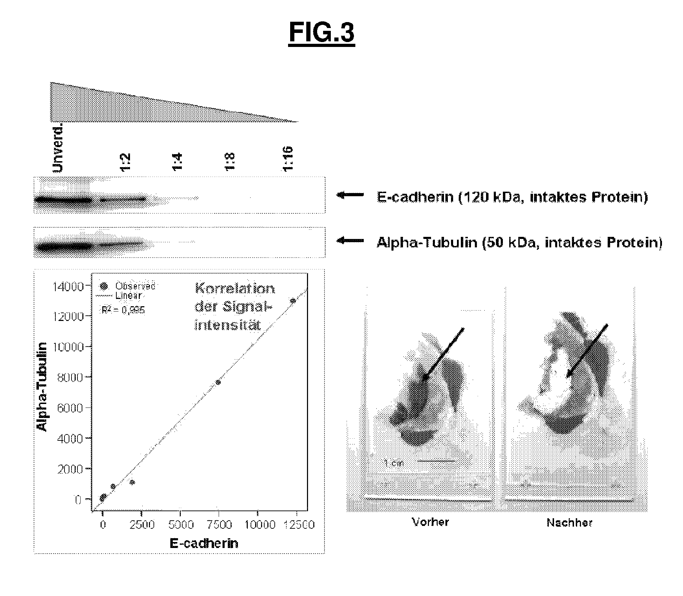

Fig.3 zeigt die Korrelation zweier Proteine (E-cadherin, alpha-Tubulin) nach Extraktion aus Formalin fixierten Geweben. Die Signalintensitäten der beiden Proteine korrelieren exzellent, d.h. intakte Proteine können nach Isolierung aus Formalin fixierten Geweben zueinander in Bezug gesetzt (quantifiziert) werden. Verschiedene Verdünnungen (1:2 bis 1:16) eines Proteinlysates von normalem Kolongewebe sind im Western Blot untersucht worden. Das Gewebe ist zur Veranschaulichung vor und nach dem Abkratzen vom Objektträger gezeigt (Pfeil). Unverd., unverdünnte Probe.

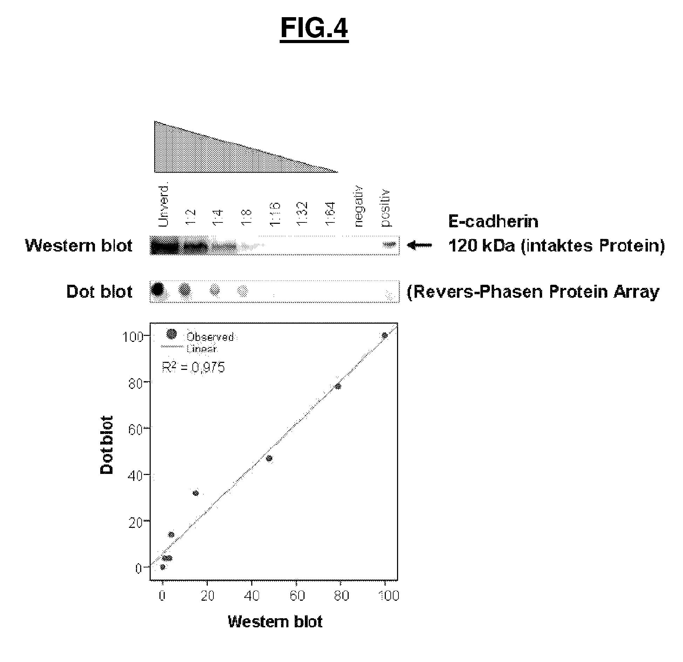

Fig.4 zeigt die Korrelation zwischen Western blot (Nachweis der Intaktheit der Proteine, Demonstration der Antikörper-Spezifität) und der einfachsten Form eines Revers-Phasen Protein Arrays (Dot blot). Verschiedene Verdünnungen (1:2 bis 1:64) eines Proteinlysates von normalem Kolongewebe sind im Western blot und Dot blot untersucht worden. Für die densitometrische Auswertung (unten) sind jeweils die Prozentwerte vergleichend zu den unverdünnten Proben aufgetragen (unverdünnte Probe = 100%). Negativ, Negativ-Kontrolle (Extraktionspuffer); positiv, Positivkontrolle (Proteinlysat aus tiefgefrorenem Kolongewebe).

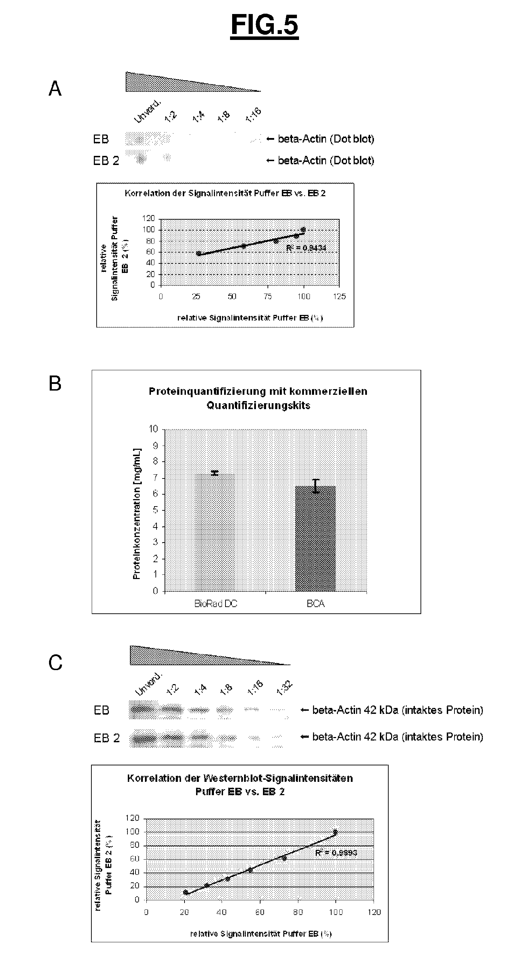

Fig.5 zeigt die Korrelation von beta-Actin nach Extraktion aus FFPE Schnitten mit Puffern, die unterschiedliche Reduktionsmittel enthalten. (A) Aus normalem Hirn-Gewebe wurden mit den beiden Extraktionspuffer EB (enthält ß-Mercapto-Ethanol) und EB 2 (enthält DTT) intakte Proteine isoliert. Verdünnungen der Proteinlysate wurden im Dot-Blot mittels ß-Actin Immunanfärbung analysiert. Die Signalintensitäten korrelieren excellent. (B) Die Proteinkonzentration des mit dem Extraktionspuffer EB 2 hergestellen Proteinlysats wurde mittels kommerziell erhältlicher Proteinquantifizierungskits bestimmt. (C) Anhand der Proteinquantifizierung mittels Proteinquantifizierungskits und der Korrelation im Dot Blot wurden je 20 µg der beiden Proteinlysate im SDS-PAGE aufgetrennt und die Menge an ß-Actin im Western-blot ermittelt. Die Signalintensitäten aus beiden Proben korrelieren exzellent und können quantifiziert werden.

Puffer bzw. Puffersystem im Sinne der Erfindung bezieht sich auf einen Puffer mit einem spezifischen pH-Wert im bereich von 1,0 bis 9,0.

Detergenzien für das erfindungsgemäße Verfahren können alle dem Fachmann bekannten und zur Zelllyse geeigneten Detergenzien sein, insbesondere werden als Detergenz SDS, Natrium-Deoxycholat, CHAPS, Triton X100, Nonidet P40 oder Tween20 verwendet.

Die Konzentration an Detergenz kann beispielsweise etwa 0.1-10% sein. Besonders bevorzugt liegt der Konzentrationsbereich zwischen etwa 1-5%.

Ferner kann der Puffer vorzugsweise zusätzlich reduzierende Reagenzien wie 1,4-Dithio-DL-threit, DTE, TCEP oder MEA umfassen.

Proteolytisch wirksame Verbindungen im Sinne der Erfindung sind alle Protein aufspaltenden Verbindungen, beispielsweise proteolytisch wirksame Enzyme wie Proteasen, insbesondere Trypsin, Chymotrypsin, Papain, Pepsin, Pronase und Endoproteinase Lys-C zu verstehen. Ferner sind proteolytisch wirksame Verbindungen im Sinne der Erfindung auch nicht-enzymatische Substanzen, die zur Spaltung von Proteinen geeignet sind, wie Bromcyan.

Biologische Proben im Sinne der Erfindung können ganze Organismen sein, Gewebeschnitte, Gewebeproben, Körperflüssigkeiten, zelluläres- oder virales Material. Vorzugsweise wird das erfindungsgemäße Verfahren bei Gewebeproben und/oder Zellkulturen verwendet. Die Proben können menschliche oder tierische biologische Proben sein, aber auch Proben aus Bakterien, Viren oder Einzellern.

Die biologischen Proben sind vorzugsweise Formalin fixiert und/oder Formalin fixiert und in Paraffin eingebettet.

Die vorliegende Erfindung beschreibt somit ein Verfahren zur Extraktion von Proteinen aus einer Formalin fixierten biologischen Probe, wobei die biologische Probe in einem Puffer bei einer Temperatur inkubiert wird, die ausreicht, um die Proteine freizusetzen, wobei der Puffer ein Detergenz und keine proteolytisch wirksame Verbindung enthält, und die biologische Probe im Puffer zunächst gekocht wird und dann bei einer Temperatur höher als 60 °C weiter inkubiert wird.

Die biologische Probe wird vorzugsweise im Puffer 5 bis 40 min gekocht. Besonders bevorzugt wird die biologische Probe für eine Dauer von 20 min bis 16 h inkubiert.

Besonderer Vorteil der vorliegenden Erfindung ist, dass die freigesetzten Proteine weitestgehend intakt sind.

Das Detergenz ist vorzugsweise SDS, Natrium-Deoxycholat, CHAPS, Triton X100, Nonidet P40 oder Tween20.

Der Puffer umfasst vorzugsweise ein zusätzliches reduzierend wirkendes Reagenz wie 1,4-Dithio-DL-threit, DTE, TCEP oder MEA.

Die biologische Probe wird, wenn es sich um eine Formalin fixierte und in Paraffin eingebettete Probe handelt, vor der Proteinextraktion entparaffiniert.

Ein Vorteil des erfindungsgemäßen Verfahrens ist, dass die extrahierten Proteine nach der Extraktion weiter fraktioniert werden können.

Insbesondere können die extrahierten Proteine mit einem oder mehreren Verfahrensschritten fraktioniert werden.

Ein weiterer Vorteil ist, dass die extrahierten Proteine anschließend quantifiziert werden können.

Die Quantifizierung der Proteine erfolgt vorzugsweise mittels der Methode nach Lowry oder BCA , wobei auch andere Quantifizierungsmethoden, insbesonders Proteinarrays, hierfür verwendet werden können.

Die extrahierten Proteine können ferner dann mittels proteolytischer Enzyme wie Trypsin, Chymotrypsin, Proteinase K, Papain, Pepsin, Pronase, Endoproteinase Lys-C, Endoproteinase Glu-C - oder Glykosidasen - wie Endoglycosidase H, N-Glycosidase F, Neuroaminidase) oder Phosphatasen behandelt werden.

Vorteilhaft ist, dass die Proteine für mindestens einen biochemischen Assay verwendet werden können.

Beispielsweise ist ein bevorzugter biochemischer Assay ein Proteinarray wie ein Mikroarray ist, insbesondere ein Sandwich Immunoarray, Antigenfängerarray oder ein direkter Proteinarray.

Der biochemische Assay kann vorzugsweise zur Bestimmung von einem oder mehreren diagnostisch oder klinisch relevanten Markerproteinen dienen.

Dabei können die Markerproteine aus mindestens zwei biologische Proben miteinander verglichen werden. Somit kann beispielsweise krank von gesund unterschieden werden. Ferner kann man den Assay auch in einem höheren Multiplexgrad durchführen um sich mindestens einen oder mehrere relevante Marker zu analysieren.

Besonders bevorzugt ist die biologische Probe eine Gewebeprobe, beispielsweise eine Formalin fixierte Gewebeprobe, die in Paraffin eingebettet ist.

Besonders bevorzugt enthält der Puffer als proteolytisch wirsame Verbindung keine Protease.

Ferner stellt die vorliegende Erfindung ein Kit zur quantitativen Extraktion von intakten Proteinen aus Formalin fixierten biologischen Proben enthaltend
(a) ein Puffersystem, das keine proteolytisch wirksame Verbindung umfasst, und
(b) ein Detergenz,
bereit.

Vorzugsweise umfasst das Kit SDS, Natrium-Deoxycholat, CHAPS, Triton X100, Nonidet P40 oder Tween20 als Detergenz.

Vorzugsweise enthält das Puffersystem als proteolytisch wirksame Verbindung keine Protease.

### Ausführungsbeispiele

### Beispiel 1: Proteinextraktion aus Formalin fixiertem Gewebe

Im Folgenden wird eine typische Proteinextraktion nach dem vorliegenden Verfahren im Detail beschrieben. Eine wesentliche Verbesserung (hohe Proteinausbeute) dieses Verfahrens im Vergleich zu herkömmlichen Methoden wird in Fig. 2 gezeigt. Der erfahrene Anwender wird den einen oder anderen Schritt des Protokolls etwas abwandeln. Es können Puffer mit etwas unterschiedlichen Zusammensetzungen und pH Werten verwendet werden. Die Verwendung eines Detergenz, z.B. SDS, das Kochen der Proben bei 95°C bis 100°C und die nachfolgende Inkubation bei wärmer 60°C (z.B. 80°C) sind jedoch entscheidend; außerdem dürfen keine Proteasen verwendet werden. Die Zeiten für die jeweilige Hitzebehandlung und die Volumina der Puffer können variieren. Ebenso variabel ist die manuelle Zerkleinerung des Gewebes. Dies kann mechanisch (z.B. mit Pistille) oder z.B. mittels Ultraschall geschehen. Typische Zeiten und Volumina sind hier aufgeführt. Das Ergebnis einer Korrelation zweier Proteine aus Formalin fixiertem Gewebe ist in Fig. 3 gezeigt. Der erste Proteinarray mit intakten Proteinen aus Formalin fixierten Geweben ist in Fig. 4 gezeigt (Revers-Phasen Proteinarray, Dot blot).

Typische Vorgehensweise:
1. 2x10 m Schnitte vom gleichen Paraffinblock herstellen
2. Schnitte entparaffinieren
2.1. 10 min Xylol, 2x wiederholen
2.2. 10 min 100% Äthanol
2.3. 10 min 96% Äthanol
2.4. 10 min 70% Äthanol
2.5. Schnitte in A. dest überführen
3. Schnitte aus A. dest nehmen, kurz antrocknen (sollten aber nicht eintrocknen)
4. Gewebeareal mit Kanüle abkratzen
5. abgekratztes Gewebe an Kanüle in 100 I Extraktionspuffer (EB)* überführen (Gewebe von 2 Schnitten pro 1.5 ml Reaktionsgefäß)
6. Gewebe in Extraktionspuffer mit Teflonpistille gut zerreiben, auf Eis stellen
7. vortexen, auf Eis stellen
8. Schritte 6 bis 7 einmal wiederholen
9. Lösung mehrmals vorsichtig durch Spritze ziehen
10. vortexen, auf Eis stellen
11. vortexen, auf Eis stellen (bei viel Schaum: kurze Zentrifugation)
12. 20 min 100°C (Wasserbad)
13. 2 Stunden 80°C (Schüttler, 750 rpm)
14. 15 min zentrifugieren, 4°C, 12500 rpm
15. Überstand in neues 1.5 ml Reaktionsgefäß -> fertiges Proteinlysat
* Herstellung von Extraktionspuffer (EB): T-PER ® (Pierce)/Lämmli 1:2
a. 5x Lämmli-Puffer Stock (ohne Bromphenolblau) 10 ml Ansatz
   2.5 ml 1.25 M Tris/HCl (pH 6.8)
   4.5 ml Glycerin
   2.8 ml beta-Mercapto-Äthanol
   1 g SDS
   ad 10 ml mit A. dest
   [1 M Tris entspr. 121.14 g ; 1.25 M entspr. 151.43 g
   1000 ml -- 151.43 g ; 50 ml -- 7.6 g; mit konz. HCl auf pH 6.8 eingestellt]
b. 2x Lämmli-Puffer: 1000 I 5x Lämmli-Puffer + 1500 I A. dest
c. Für 5 ml Extraktionspuffer:
   2.5 ml T-PER ® (Pierce)
   + 2.5 ml 2xLämmli-Puffer
   + ½ complete Mini Protease-Inhibitor-Tablette (Bayer)
Aliquots bei -20°C einfrieren

### Beispiel 2: Proteinextraktion aus FFPE Gewebe mit alternativem Extraktionspuffer EB2

In einer weiteren Ausführungsform des erfinderischen Verfahrens wird DTT (1,4-Dithio-DL-threit) verwendet. DTT wird bevorzugt verwendet bei der Quantifizierung der isolierten Proteine, beispielsweise mittels dem DC Protein Assay Kit® der Firma Bio-Rad oder dem Micro BCA Protein Assay Kit ® von Pierce (Fig. 5).

Die SDS-Konzentration und die entsprechenden Inkubationszeiten bei 100°C bzw. 80°C wurden nicht modifiziert. Vorteil dieser Ausführungsform ist, dass die Konzentration fertiger Protein-Lysate direkt mit einem kommerziell verfügbaren Proteinquantifizierungskit bestimmt werden kann, ohne dass Komponenten - hier speziell das Reduktionsmittel - mit dem Assay interferieren könnten. Die Proteinkonzentration kann besser im linearen Messbereich gemessen werden, ggf. muss die Probe dann nur aufgrund eines zu hohen Proteingehaltes verdünnt werden. Auf diese Weise wird ein noch genaueres Messergebnis erhalten und für Downstream-Applikationen kann somit die entsprechende Proteinmenge eingesetzt werden.
1. 2x10 m Schnitte vom gleichen Paraffinblock schneiden und in ein Reaktionsgefäß überführen
2. Schnitte entparaffinieren
   2.1. 10 min Xylol, 2x wiederholen
   2.2. 10 min 100% Äthanol
   2.3. 10 min 96% Äthanol
   2.4. 10 min 70% Äthanol
3. 100 I Extraktionspuffer (EB2)* zugeben
4. vortexen, auf Eis stellen,
5. Lösung mehrmals vorsichtig mit Pipette aufnehmen
6. vortexen, auf Eis stellen (bei viel Schaum: kurze Zentrifugation)
7. 20 min 100°C (Wasserbad)
8. 2 Stunden 80°C (Schüttler, 750 rpm)
9. 15 min zentrifugieren, 4°C, 12500 rpm
10. Überstand in ein frisches 1.5 ml Reaktionsgefäß überführen → fertiges Proteinlysat
11. Quantifizieren mittels kommerzieller Proteinquantifizierungskits
Alternativer Extraktionspuffer 2 (EB2)
90 mM Tris/HCl (pH 6.8)
20 % Glycerin
2%SDS
1 mM DTT (1,4-Dithio-DL-threit)
Aliquots bei -20°C einfrieren

## Patentansprüche

1. Verfahren zur Extraktion von Proteinen aus einer Formalin fixierten biologischen Probe, wobei die biologische Probe in einem Puffer bei einer Temperatur inkubiert wird, die ausreicht, um die Proteine freizusetzen, **dadurch gekennzeichnet, dass** der Puffer ein Detergenz und keine proteolytisch wirksame Verbindung enthält, und die biologische Probe im Puffer zunächst gekocht und dann bei einer Temperatur höher als 60 °C weiter inkubiert wird.

2. Verfahren nach Anspruch 1, wobei die biologische Probe im Puffer 5 bis 40 min gekocht wird.

3. Verfahren nach Anspruch 1 oder 2, wobei die biologische Probe für eine Dauer von 20 min bis 16 h inkubiert wird.

4. Verfahren nach einem der Ansprüche 1 bis 3, wobei das Detergenz SDS, Natrium-Deoxycholat, CHAPS, Triton X100, Nonidet P40 oder Tween20 ist.

5. Verfahren nach einem der Ansprüche 1 bis 4, wobei der Puffer zusätzlich reduzierende Reagenzien wie 1,4-Dithio-DL-threit, DTE, TCEP oder MEA umfasst.

6. Verfahren nach einem der Ansprüche 1 bis 5, wobei die biologische Probe, wenn es sich um eine Formalin fixierte und in Paraffin eingebettete Probe handelt, vor der Proteinextraktion entparaffiniert wird.

7. Verfahren nach einem der Ansprüche 1 bis 6, wobei die Proteine nach der Extraktion weiter fraktioniert werden.

8. Verfahren nach Anspruch 7, wobei die extrahierten Proteine mit einem oder mehreren Verfahrensschritten fraktioniert werden.

9. Verfahren nach einem der Ansprüche 1 bis 8, wobei die extrahierten Proteine anschließend quantifiziert werden.

10. Verfahren nach Anspruch 9, wobei die Quantifizierung der Proteine mittels der Methode nach Lowry oder BCA erfolgt.

11. Verfahren nach einem der Ansprüche 1 bis 10, wobei die extrahierten Proteine mittels proteolytischer Enzyme wie Trypsin, Chymotrypsin, Proteinase K, Papain, Pepsin, Pronase, Endoproteinase Lys-C, Endoproteinase Glu-C - oder Glykosidasen - wie Endoglycosidase H, N-Glycosidase F, Neuroaminidase) oder Phosphatasen behandelt werden.

12. Verfahren nach einem der Ansprüche 1 bis 11, wobei die Proteine für mindestens einen biochemischen Assay verwendet werden.

13. Verfahren nach Anspruch 12, wobei der biochemische Assay ein Proteinarray wie ein Mikroarray ist, insbesondere ein Sandwich Immunoarray, Antigenfängerarray oder ein direkter Proteinarray.

14. Verfahren nach Anspruch 12 oder 13 wobei der biochemische Assay zur Bestimmung von einem oder mehreren diagnostisch oder klinisch relevanten Markerproteinen dient.

15. Verfahren nach Anspruch 14, wobei die Markerproteine aus mindestens zwei biologische Proben miteinander verglichen werden.

16. Verfahren nach einem der Ansprüche 1 bis 15, wobei die biologische Probe eine Gewebeprobe ist.

17. Verfahren nach einem der Ansprüche 1 bis 16, wobei die proteolytisch wirksame Verbindung eine Protease ist.

## Claims

1. Method for extracting proteins from a formalin-fixed biological sample, wherein the biological sample is incubated in a buffer at a temperature sufficient to release the proteins, **characterized in that** the buffer contains a detergent and no proteolytically active compound, and the biological sample is firstly boiled in the buffer and then further incubated at a temperature greater than 60°C.

2. Method according to Claim 1, wherein the biological sample is boiled in the buffer for 5 to 40 min.

3. Method according to Claim 1 or 2, wherein the biological sample is incubated for a period of from 20 min to 16 h.

4. Method according to any of Claims 1 to 3, wherein the detergent is SDS, sodium deoxycholate, CHAPS, Triton X100, Nonidet P40 or Tween 20.

5. Method according to any of Claims 1 to 4, wherein the buffer additionally comprises reducing agents such as 1,4-dithio-DL-threitol, DTE, TCEP or MEA.

6. Method according to any of Claims 1 to 5, wherein the biological sample, if it is a sample which is formalin-fixed and embedded in paraffin, is deparaffinized before the protein extraction.

7. Method according to any of Claims 1 to 6, wherein the proteins are further fractionated after the extraction.

8. Method according to Claim 7, wherein the extracted proteins are fractionated using one or more method steps.

9. Method according to any of Claims 1 to 8, wherein the extracted proteins are subsequently quantified.

10. Method according to Claim 9, wherein the proteins are quantified using the Lowry or BCA method.

11. Method according to any of Claims 1 to 10, wherein the extracted proteins are treated using proteolytic enzymes such as trypsin, chymotrypsin, proteinase K, papain, pepsin, pronase, endoproteinase Lys-C, endoproteinase Glu-C - or glycosidases - such as endoglycosidase H, N-glycosidase F, neuraminidase or phosphatases.

12. Method according to any of Claims 1 to 11, wherein the proteins are used for at least one biochemical assay.

13. Method according to Claim 12, wherein the biochemical assay is a protein array such as a microarray, more particularly a sandwich immunoarray, antigen capture array or a direct protein array.

14. Method according to Claim 12 or 13, wherein the biochemical assay is used for determining one or more diagnostically or clinically relevant marker proteins.

15. Method according to Claim 14, wherein the marker proteins from at least two biological samples are compared with one another.

16. Method according to any of Claims 1 to 15, wherein the biological sample is a tissue sample.

17. Method according to any of Claims 1 to 16, wherein the proteolytically active compound is a protease.

## Revendications

1. Procédé pour l'extraction de protéines à partir d'un échantillon biologique fixé avec une solution de formaldéhyde, dans lequel on met l'échantillon biologique à incuber dans un tampon à une température qui est suffisante pour libérer les protéines, **caractérisé en ce que** le tampon contient un détergent et ne contient aucun composé à activité protéolytique, et on fait d'abord bouillir l'échantillon biologique dans le tampon, et ensuite on le met de nouveau à incuber à une température supérieure à 60 °C.

2. Procédé selon la revendication 1, dans lequel on fait bouillir l'échantillon biologique dans le tampon pendant 5 à 40 min.

3. Procédé selon la revendication 1 ou 2, dans lequel on met l'échantillon biologique à incuber pendant une durée de 20 min à 16 h.

4. Procédé selon l'une quelconque des revendications 1 à 3, dans lequel le détergent est le SDS, le désoxycholate de sodium, le CHAPS, le Triton X100, le Nonidet P40 ou le Tween 20.

5. Procédé selon l'une quelconque des revendications 1 à 4, dans lequel le tampon comprend en outre des réactifs réducteurs tels que le 1,4-dithio-DL-thréitol, le DTE, la TCEP, ou la MEA.

6. Procédé selon l'une quelconque des revendications 1 à 5, dans lequel l'échantillon biologique, lorsqu'il s' agit d'un échantillon fixé avec une solution de formaldéhyde et inclus dans de la paraffine, est déparaffiné avant l'extraction des protéines.

7. Procédé selon l'une quelconque des revendications 1 à 6, dans lequel après l'extraction on soumet les protéines à un fractionnement plus poussé.

8. Procédé selon la revendication 7, dans lequel on fractionne à l'aide d'une ou de plusieurs étapes de processus les protéines extraites.

9. Procédé selon l'une quelconque des revendications 1 à 8, dans lequel les protéines extraites sont ensuite quantifiées.

10. Procédé selon la revendication 9, dans lequel la quantification des protéines s'effectue par la méthode de Lowry ou BCA.

11. Procédé selon l'une quelconque des revendications 1 à 10, dans lequel les protéines extraites sont traitées par des enzymes protéolytiques telles que la trypsine, la chymotrypsine, la protéinase K, la papaïne, la pepsine, la pronase, l'endoprotéinase Lys-C, l'endoprotéinase Glu-C - ou des glycosidases - telles que l'endoglycosidase H, la N-glycosidase F, la neuroaminidase ou des phosphatases.

12. Procédé selon l'une quelconque des revendications 1 à 11, dans lequel on utilise les protéines pour au moins un essai biochimique.

13. Procédé selon la revendication 12, dans lequel l'essai biochimique est un réseau de protéines, tel qu'un microréseau, en particulier un immunoréseau en sandwich, un réseau de capteurs d'antigènes ou un réseau de protéines direct.

14. Procédé selon la revendication 12 ou 13, dans lequel l'essai biochimique sert à la détermination d'une ou de plusieurs protéines de marquage d'intérêt diagnostique ou clinique.

15. Procédé selon la revendication 14, dans lequel on compare entre elles les protéines de marquage provenant d'au moins deux échantillons biologiques.

16. Procédé selon l'une quelconque des revendications 1 à 15, dans lequel l'échantillon biologique est un échantillon de tissu.

17. Procédé selon l'une quelconque des revendications 1 à 16, dans lequel le composé à activité protéolytique est une protéase.
